# EUROPÄISCHE PATENTSCHRIFT

(11) **EP 3 722 804 B1**
(45) Veröffentlichungstag und Bekanntmachung des Hinweises auf die Patenterteilung: **23.08.2023**
(21) Anmeldenummer: 20164788.0
(22) Anmeldetag: 23.03.2020
(51) Int. Cl.: G01N 33/24, G01N 22/04

(54) **VERFAHREN UND SYSTEM ZUR MESSUNG DER BODENFEUCHTE**
METHOD AND SYSTEM FOR MEASURING GROUND MOISTURE
PROCÉDÉ ET SYSTÈME DE MESURE DE L'HUMIDITÉ DU SOL

(30) Priorität: 10.04.2019 DE 102019205117
(43) Veröffentlichungstag der Anmeldung: 14.10.2020
(73) Patentinhaber: Deutsches Zentrum für Luft- und Raumfahrt e.V., 51147 Köln (DE)
(72) Erfinder: Thiel, Christian, 07751 Jena (DE); Truckenbrodt, John, 07743 Jena (DE)
(74) Vertreter: Patentanwälte Bressel und Partner mbB

(56) Entgegenhaltungen:
- US-A- 3 553 573
- US-A1- 2017 108 452
- XIN DONG ET AL: "Autonomous precision agriculture through integration of wireless underground sensor networks with center pivot irrigation systems", AD HOC NETWORKS, Bd. 11, Nr. 7, 20. Juli 2012 (2012-07-20), Seiten 1975-1987, XP055725336, AMSTERDAM, NL ISSN: 1570-8705, DOI: 10.1016/j.adhoc.2012.06.012
- H.R. BOGENA ET AL: "Potential of Wireless Sensor Networks for Measuring Soil Water Content Variability", VADOSE ZONE JOURNAL, Bd. 9, Nr. 4, 7. September 2010 (2010-09-07), Seiten 1002-1013, XP055725417, US ISSN: 1539-1663, DOI: 10.2136/vzj2009.0173

## Beschreibung

Die Erfindung betrifft ein Verfahren und ein System zur Messung der Bodenfeuchte.

Die oberflächennahe Bodenfeuchte ist ein entscheidender Umweltparameter. Sie steuert Prozesse wie Infiltration, Oberflächenabfluss sowie Evaporation und reguliert das Verhältnis zwischen spürbarer und latenter Wärme. Somit stellt dieser Parameter einen wertvollen Input für ereignisbasierte hydrologische Modelle, für Hochwasservorhersagemodelle, für die Erosionsmodellierung sowie für Stofftransportmodelle dar. Zudem besitzt er ausschlaggebenden Charakter in Klima- und globalen Zirkulationssimulationen.

Die Bodenfeuchte kann anhand der dielektrischen Eigenschaften des Bodens bestimmt werden. Die dielektrischen Eigenschaften werden durch die komplexe Dielektrizitätskonstante, welche einen realen (Durchlässigkeit des Materials) sowie einen imaginären (Absorption durch das Medium) Anteil besitzt, ausgedrückt. Die komplexe Dielektrizitätskonstante wirkt demnach als Steuergröße für die Reflexion bzw. Refraktion, die Absorption sowie die Änderung der Ausbreitungsrichtung einer elektromagnetischen (EM) Welle. Für bestimmte Stoffe ist die Dielektrizitätskonstante, die experimentell bestimmt wurde, aus entsprechenden Tafeln zu entnehmen. Böden stellen allerdings ein Stoffgemisch dar. In diesem Fall resultiert die Dielektrizitätskonstante aus der stofflichen Zusammensetzung des Bodens. Die wesentlichen Bestandteile sind mineralische Partikel, organische Anteile, Bodenluft sowie Bodenwasser. Das Bodenwasser besitzt aufgrund der hohen Dielektrizitätskonstante von Wasser den größten Einfluss auf die dielektrischen Eigenschaften des Bodens. Die Dielektrizitätskonstante eines Bodens nimmt mit steigende Bodenfeuchte zu. Für jeden Boden lässt sich ein empirischer Zusammenhang zwischen der Dielektrizitätskonstante und der Bodenfeuchte finden.

Für die Messung der dielektrischen Eigenschaften wurden bereits einige Verfahren entwickelt. Die Messung der Dielektrizitätskonstante kann beispielsweise auf Basis der Time Domain Reflectometry (TDR) erfolgen. Dieses Verfahren basiert auf der Abhängigkeit der Ausbreitungsgeschwindigkeit eines elektromagnetischen Pulses von der elektrischen Kapazität und somit der Dielektrizitätskonstante eines Mediums. Ein weiteres Verfahren stellt die Frequency Domain Reflectometry (FDR) dar. Das Instrument verfügt über einen Schwingkreis, der Sensorteil des Sensors ist in den Boden eingebettet und die Betriebsfrequenz hängt von dem Wert der Dielektrizitätskonstante des Bodens ab. Zudem existiert eine Reihe ähnlicher Verfahren.

Ein großer Nachteil bereits bestehender Messverfahren der Dielektrizitätskonstante eines Bodens bzw. der Bodenfeuchte besteht darin, dass diese Systeme sehr komplex sind hinsichtlich Hard- und Software, da hochpräzise Messungen beispielsweise der Laufzeiten der EM Wellen erforderlich sind. Dies führt zu hohen Herstellungskosten und entsprechend hohen Gerätepreisen. Dementsprechend ist eine flächenhafte Verbauung derartiger Sensorik zur Regionalisierung von Bodenfeuchtemessungen nur mit großem Aufwand möglich.

Xin Dong et al.: "Autonomous precision agriculture through integration of wireless underground sensor networks with center pivot irrigation systems", AD HOC NETWORKS, Bd. 11, Nr. 7, 20. July 2012, Seiten 1975-1987 und H. R. Bogena et al.: Potential of Wireless Sensor Networks for Measuring Soil Water Content Variability", VADOSE ZONE JOURNAL, Bd. 9, Nr. 4, 7. September 2010, Seiten 1002-1013 offenbaren jeweils Sensor-Netzwerke zur Ermittlung der Bodenfeuchte. Dabei erfassen kapazitive Sensoren im Boden die lokale Bodenfeuchte, wobei die Messdaten ausgetauscht werden und von bestimmten Sensoren an die Erdoberfläche zu einem Empfänger übertragen werden.

Aus der US 3,553,573 A ist ein Verfahren bekannt, bei dem mittels Mikrowellen die Feuchtigkeit von granulatförmigen, zellulose-haltigen Materialien bestimmt wird.

Aus der US 2017/0108452 A1 ist ein Time-of-Flight-Sensor bekannt, wobei aus den Messdaten mindestens eine Eigenschaft eines Mediums aufgrund der Änderung der Dielektrizitätskonstanten ermittelt wird.

Der Erfindung liegt daher das technische Problem zugrunde, ein vereinfachtes Verfahren zur Messung der Bodenfeuchte zur Verfügung zu stellen sowie ein hierfür geeignetes System zu schaffen.

Die Lösung des technischen Problems ergibt sich durch ein Verfahren mit den Merkmalen des Anspruchs 1 sowie eines Systems mit den Merkmalen des Anspruchs 5. Weitere vorteilhafte Ausgestaltungen der Erfindung ergeben sich aus den Unteransprüchen.

Das Verfahren zur Messung der Bodenfeuchte erfolgt mittels mindestens eines Senders und eines Empfängers im Mikrowellenbereich zwischen 300 MHz bis 300 GHz sowie einer Auswerteeinheit. Dabei strahlt der mindestens eine im Boden verbaute Sender eine definierte Sendeleistung ab, wobei eine Empfangsleistung an dem mindestens einen Empfänger empfangen wird, wobei sich der Empfänger oberhalb des Bodens befindet. Die Auswerteeinheit ermittelt dann die Bodenfeuchte unter Berücksichtigung der Einbautiefe des Senders, der Höhe des Empfängers über dem Boden, der Sende- und Empfangsleistung, der Frequenz des Senders sowie mindestens einem Referenzwert des Bodens die aktuelle Bodenfeuchte. Hierdurch wird ein sehr einfaches und robustes Verfahren zur Ermittlung der Bodenfeuchte zur Verfügung gestellt. Dabei wird ausgenutzt, dass Mikrowellen die Fähigkeit besitzen, Medien in Abhängigkeit von deren Dielektrizitätskonstante zu durchdringen. Mit steigender Dielektrizitätskonstante erfolgt eine zunehmende Dämpfung des Signals. Die absolute Dämpfung des Signals hängt dabei überwiegend von der Dielektrizitätskonstanten des Bodens sowie von der Laufstrecke durch den Boden ab. Die Referenzwerte für den Boden werden dabei vorzugsweise vorab empirisch ermittelt und spiegeln den Zusammenhang der Dielektrizitätskonstanten des Bodens zur Bodenfeuchte wider.

In einer Ausführungsform des Verfahrens ermittelt der Empfänger seine Höhe zum Boden. Dies kommt insbesondere zum Einsatz, wenn der Empfänger ein mobiler Empfänger gemäß des Systems der Erfindung ist, der beispielsweise von einem Nutzer gehalten wird. In diesem Fall ermittelt dann der Empfänger mittels geeigneter Sensorik seine Höhe selbst. Bei stationären Lösungen des Empfängers ist dies eine Konstante.

In einer weiteren Ausführungsform des Verfahrens und im erfindungsgemäßen System überträgt der Sender seine Einbautiefe und/oder Sendeleistung codiert im Sendesignal. Beispielsweise ist das Sendesignal ein PWM-Signal, wobei das Tastverhältnis die Sendeleistung und/oder Einbautiefe widerspiegelt. Dabei können aber auch ein oder beide Parameter als Konstante dem Empfänger bekannt sein.

In einer weiteren Ausführungsform sendet der Sender im Bereich von 2,402 GHz bis 2,480 GHz. Der Vorteil liegt darin, dass dies die Frequenz des lizenzfreien ISB-Bandes ist (Industrial, Scientific and Medical Band). Aufgrund der großen Wellenlänge im Zusammenspiel mit Klasse 1 Sendern (100 mW) sind damit Einbautiefen von ca. 10 cm möglich. Die Frequenz kann vom Empfänger bestimmt werden oder aber auch vorbekannt sein.

Hinsichtlich der Ausgestaltung von Sender und Empfänger wird auf die vorangegangenen Ausführungen Bezug genommen.

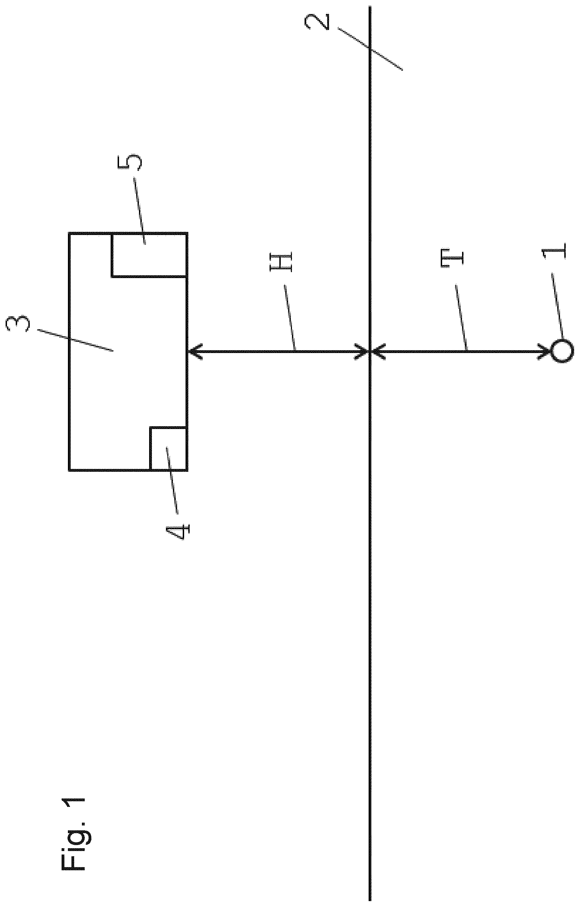

Die Erfindung wird nachfolgend anhand eines bevorzugten Ausführungsbeispiels näher erläutert. Die einzige Figur zeigt eine schematische Darstellung einer Anordnung von Sender und Empfänger zur Messung der Bodenfeuchte.

Ein Sender 1 für Mikrowellen ist im Boden 2 in einer Einbautiefe T verbaut. Der Sender 1 weist eine Batterie auf und ist beispielsweise als Bluetooth Low Energy Beacon (BLE) ausgebildet. Über dem Boden 2 in einer Höhe H ist ein Empfänger 3 angeordnet, der zusätzlich eine Auswerteeinheit 4 und eine Sensorik 5 zur Erfassung der Höhe H aufweist. Der Empfänger 3 empfängt nun ein Empfangssignal vom Sender 1 und ermittelt die Empfangsleistung und die Höhe H. Der Auswerteeinheit 4 wird nun die Empfangsleistung und die Höhe H zugeführt. Des Weiteren kennt die Auswerteeinheit 4 die Frequenz des Senders 1, dessen Einbautiefe T, die Sendeleistung des Senders und einen Referenzwert für die Dielektrizitätskonstanten des Bodens 2. Diese Parameter können a priori festgelegt sein oder aber werden, in einem System gemäß der Erfindung, im Sendesignal codiert mitgeteilt.

Anhand dieser Parameter kann dann die Auswerteeinheit die aktuelle Dielektrizitätskonstante des Bodens 2 ermitteln und daraus auf die aktuelle Bodenfeuchte zurückrechnen.

Der Empfänger 3 des Systems gemäß der Erfindung ist als mobiles Endgerät wie beispielsweise ein Smart-Phone ausgebildet.

Das Auffinden des eingegrabenen Senders 1 kann auf verschiedene Arten erfolgen.

Entweder ist auf dem Boden 2 eine Markierung aufgebracht oder aber der Empfänger hat eine Karte, auf der die Positionen der Sender 1 eingezeichnet sind, wobei durch entsprechende Lokalisierungen wie beispielsweise GPS sich der Empfänger 3 zum Sender 1 lokalisiert.

## Patentansprüche

1. Verfahren zur Messung der Bodenfeuchte, mittels mindestens eines Senders (1) und eines Empfängers (3) im Mikrowellenbereich zwischen 300 MHz bis 300 GHz sowie einer Auswerteeinheit (4), umfassend die folgenden Verfahrensschritte:
a) Abstrahlen einer definierten Sendeleistung durch den mindestens einen im Boden (2) verbauten Sender (1),
b) Empfangen einer Empfangsleistung an dem mindestens einen Empfänger (3) und
c) Ermitteln einer Bodenfeuchte in der Auswerteeinheit (4) unter Berücksichtigung der Einbautiefe (T) des Senders (1), der Höhe (H) des Empfängers (3) über dem Boden (2), der Sende- und Empfangsleistung, der Frequenz sowie mindestens einem Referenzwert des Bodens (2).

2. Verfahren nach Anspruch 1, **dadurch gekennzeichnet, dass** der Empfänger (3) seine Höhe (H) zum Boden (2) ermittelt.

3. Verfahren nach Anspruch 1 oder 2, **dadurch gekennzeichnet, dass** der Sender (1) seine Einbautiefe (T) und/oder Sendeleistung im Sendesignal codiert überträgt.

4. Verfahren nach einem der vorangegangenen Ansprüche, **dadurch gekennzeichnet, dass** der Sender (1) im Bereich von 2,402 GHz bis 2,480 GHz sendet.

5. System zur Messung der Bodenfeuchte, umfassend mindestens einen Sender (1) und einen Empfänger (3), wobei der Sender (1) derart ausgebildet ist, dass dieser ein definiertes Signal im Mikrowellenbereich zwischen 300 MHz bis 300 GHz emittiert, wobei der Sender (1) derart ausgebildet ist, seine Sendeleistung codiert im Sendesignal zu übertragen, wobei der Empfänger (3) derart ausgebildet ist, ein definiertes Signal im Mikrowellenbereich zwischen 300 MHz bis 300 GHz zu empfangen und die Empfangsleistung zu ermitteln, wobei der Empfänger (3) eine Auswerteeinheit (4) und eine Sensorik (5) zur Erfassung der Höhe (H) aufweist, wobei die Auswerteeinheit (4) derart ausgebildet ist, unter Berücksichtigung der Einbautiefe (T) eines Senders (1), der Höhe (H) des Empfängers über einem Boden (2), der Sende- und Empfangsleistung einer Frequenz des Senders (1) sowie mindestens einem Referenzwert des Bodens (2) eine Bodenfeuchte zu ermitteln, wobei der Empfänger (3) als mobile Einheit ausgebildet ist, wobei die Sendeleistung aus dem empfangenen Signal decodiert wird.

6. System nach Anspruch 5, **dadurch gekennzeichnet, dass** der Sender (1) derart ausgebildet ist, seine Einbautiefe (T) codiert im Sendesignal zu übertragen.

7. System nach Anspruch 5 oder 6, **dadurch gekennzeichnet, dass** der Sender (1) eine Batterie als Energieversorgung aufweist

8. System nach einem der Ansprüche 5 bis 7, **dadurch gekennzeichnet, dass** der Sender (1) derart ausgebildet ist, im Bereich von 2,402 GHz bis 2,480 GHz zu senden.

## Claims

1. Method for measuring ground moisture, by means of at least one transmitter (1) and a receiver (3) in the microwave range between 300 MHz and 300 GHz and an evaluation unit (4), comprising the following method steps:
a) emitting a defined transmitted power by way of the at least one transmitter (1) installed on the ground (2),
b) receiving a received power at the at least one receiver (3), and
c) ascertaining a ground moisture in the evaluation unit (4) by taking account of the installation depth (T) of the transmitter (1), the height (H) of the receiver (3) above the ground (2), the transmitted and received powers, the frequency and at least one reference value for the ground (2).

2. Method according to Claim 1, **characterized in that** the receiver (3) ascertains its height (H) in relation to the ground (2).

3. Method according to Claim 1 or 2, **characterized in that** the transmitter (1) transmits its installation depth (T) and/or transmitted power in the transmission signal in coded form.

4. Method according to one of the preceding claims, **characterized in that** the transmitter (1) transmits in the range from 2.402 GHz to 2.480 GHz.

5. System for measuring ground moisture, comprising at least one transmitter (1) and a receiver (3), wherein the transmitter (1) is designed in such a way that it emits a defined signal in the microwave range between 300 MHz and 300 GHz, wherein the transmitter (1) is designed to transmit its transmitted power in coded form in the transmission signal, wherein the receiver (3) is designed to receive a defined signal in the microwave range between 300 MHz and 300 GHz and to ascertain the received power, wherein the receiver (3) has an evaluation unit (4) and a sensor system (5) for recording the height (H), wherein the evaluation unit (4) is designed to take account of the installation depth (T) of a transmitter (1), the height (H) of the receiver above a ground (2), the transmitted and received powers, a frequency of the transmitter (1) and at least one reference value for the ground (2) in order to ascertain a ground moisture, wherein the receiver (3) is in the form of a mobile unit, wherein the transmitted power is decoded from the received signal.

6. System according to Claim 5, **characterized in that** the transmitter (1) is designed to transmit its installation depth (T) in coded form in the transmission signal.

7. System according to Claim 5 or 6, **characterized in that** the transmitter (1) has a battery as power supply.

8. System according to one of Claims 5 to 7, **characterized in that** the transmitter (1) is designed to transmit in the range from 2.402 GHz to 2.480 GHz.

## Revendications

1. Procédé de mesure de l'humidité du sol, au moyen d'au moins un émetteur (1) et un récepteur (3) dans la gamme des hyperfréquences entre 300 MHz et 300 GHz ainsi que d'une unité d'interprétation (4), comprenant les étapes suivantes :
a) rayonnement d'une puissance d'émission définie par l'au moins un émetteur (1) monté dans le sol (2),
b) réception d'une puissance de réception au niveau de l'au moins un récepteur (3) et
c) détermination d'un taux d'humidité du sol dans l'unité d'interprétation (4) en tenant compte de la profondeur de pose (T) de l'émetteur (1), de la hauteur (H) du récepteur (3) au-dessus du sol (2), de la puissance d'émission et de réception, de la fréquence ainsi que d'au moins une valeur de référence du sol (2).

2. Procédé selon la revendication 1, **caractérisé en ce que** le récepteur (3) détermine sa hauteur (H) par rapport au sol (2).

3. Procédé selon la revendication 1 ou 2, **caractérisé en ce que** l'émetteur (1) transmet sa profondeur de pose (T) et/ou sa puissance d'émission sous forme codée dans le signal émis.

4. Procédé selon l'une des revendications précédentes, **caractérisé en ce que** l'émetteur (1) émet dans la plage de 2,402 GHz à 2,480 GHz.

5. Système de mesure de l'humidité du sol, comprenant au moins un émetteur (1) et un récepteur (3), l'émetteur (1) étant configuré de telle sorte que celui-ci émet un signal défini dans la gamme des hyperfréquences entre 300 MHz et 300 GHz, l'émetteur (1) étant configuré de manière à transmettre sa puissance d'émission sous forme codée dans le signal émis, le récepteur (3) étant configuré de manière à recevoir un signal défini dans la gamme des hyperfréquences entre 300 MHz et 300 GHz et déterminer la puissance de réception, le récepteur (3) possédant une unité d'interprétation (4) et un système de détection (5) destiné à capter la hauteur (H), l'unité d'interprétation (4) étant configurée pour déterminer un taux d'humidité du sol en tenant compte de la profondeur de pose (T) d'un émetteur (1), de la hauteur (H) du récepteur (3) au-dessus d'un sol (2), de la puissance d'émission et de réception d'une fréquence de l'émetteur (1) ainsi que d'au moins une valeur de référence du sol (2), le récepteur (3) étant réalisé sous la forme d'une unité mobile, la puissance d'émission étant décodée à partir du signal reçu.

6. Système selon la revendication 5, **caractérisé en ce que** l'émetteur (1) est configuré pour transmettre sa profondeur de pose (T) sous forme codée dans le signal émis.

7. Système selon la revendication 5 ou 6, **caractérisé en ce que** l'émetteur (1) possède une pile/batterie en tant que source d' énergie.

8. Système selon l'une des revendications 5 à 7, **caractérisé en ce que** l'émetteur (1) est configuré pour émettre dans la plage de 2,402 GHz à 2,480 GHz.
